# EUROPEAN PATENT APPLICATION

(11) **EP 2 498 195 A1**
(43) Date of publication of application: **12.09.2012**
(21) Application number: 11157978.5
(22) Date of filing: 11.03.2011
(51) Int. Cl.: G06F 19/00

(54) **Non-invasive method for assessing the presence or severity of liver fibrosis based on a new detailed classification**

(71) Applicant: Centre Hospitalier Universitaire d'Angers, 49100 Angers (FR); UNIVERSITE D'ANGERS, 49000 Angers (FR)
(72) Inventor: Calès, Paul, 49240, Avrillé (FR); Boursier, Jérôme, 49000, Angers (FR)
(74) Representative: Icosa

(57) **Abstract**

The present invention relates to a non-invasive method for assessing the presence and/or severity of a lesion in an organ of an animal, including a human, said method comprising carrying out at least one non-invasive test resulting in a score/value, and positioning the at least one score/value in a class of a detailed classification based on population percentiles, or in a reliable diagnostic interval (RDI), to be crossed with another RDI for a final positioning of both RDI in a class. The present invention also relates to a device, preferably a meter, carrying out the non-invasive method of the invention.

## Description

### FIELD OF INVENTION

The present invention relates to a non-invasive method for assessing the presence and/or the severity of liver fibrosis or cirrhosis. More specifically, the present invention relates to a non-invasive method implementing a new detailed classification of liver fibrosis stages leading to an improved diagnostic accuracy and precision.

### BACKGROUND OF INVENTION

Liver fibrosis refers to the accumulation in the liver of fibrous scar tissue in response to injury of the hepatocytes due to various etiologies, such as for examples infection with a virus (such as hepatitis viruses HCV and HBV), heavy alcohol consumption, toxins or trauma. The evolution of the fibrosis phenomena may lead to cirrhosis, a condition in which the ability of the liver to function is impaired. Treatments of liver fibrosis exist, which can slow or halt fibrosis progression, and even reverse existing liver damages. On the contrary, cirrhosis is usually non reversible. Therefore, the earlier the diagnostic of a fibrosis is, the more elevated the chances of reversion are.

Liver biopsy is the historical means in order to diagnose liver diseases in patients. Various systems, based on liver biopsies, are used to grade fibrosis and cirrhosis, such as, for example, Metavir and Ishak (where cirrhosis is graded). Using Metavir scoring system for fibrosis, five classes (named Metavir **F** stages) are distinguished: F0 (no fibrosis, no scarring), F1 (portal fibrosis, minimal scarring), F2 (few septa, scarring has occurred and extents outside the areas in the liver that contains blood vessels), F3 (many septa, bridging fibrosis is spreading and connecting to other areas that contain fibrosis) and finally F4 (cirrhosis or advanced scarring of the liver). Fibrosis of stages F3 or F4 are considered as "severe fibrosis". For patients with "clinically significant fibrosis" (i.e. with Metavir score ≥ F2), a treatment is usually recommended, whereas patients with no or mild fibrosis (F0 or F1 Metavir score) do not usually receive any treatment, but are monitored for fibrosis progression. Ranging a patient according to the Metavir classification helps for determining the adapted treatment for said patient. In this patent application, any citation of F0, F1, F2, F3 and F4 is made with reference to the Metavir stages.

When using Metavir scoring system for assessing necrotico-inflammatory activity, four grades (named Metavir A grades) are distinguished: A0 (absence of necrotico-inflammatory activity), A1 (low necrotico-inflammatory activity), A2 (moderate necrotico-inflammatory activity), and A3 (high necrotico-inflammatory activity). In this patent application, any citation of A0, A1, A2, A3 is made with reference to the Metavir grades.

However, since liver biopsy is invasive and expensive, non-invasive diagnosis of liver fibrosis has gained considerable attention over the last 10 years as an alternative to liver biopsy. The first generation of simple blood fibrosis tests combined common indirect blood markers into a simple ratio, like APRI (Wai et al., Hepatology 2003) or FIB-4 (Sterling et al., Hepatology 2006). The second generation of calculated tests combined indirect and/or direct fibrosis markers by logistic regression, leading to a score, like Fibrotest (Imbert-Bismut et al., Lancet 2001), ELF score (Rosenberg et al., Gastroenterology 2004), FibroMeter™ (Cales et al., Hepatology 2005), Fibrospect™ (Patel et al., J Hepatol 2004), and Hepascore (Adams et al., Clin Chem 2005). For example, WO2005/116901 describes a non-invasive method for assessing the presence of a liver disease and its severity, by measuring levels of specific variables, including biological variables and clinical variables, and combining said variables into mathematical functions to provide a score, often called "score of fibrosis".

For statistical reasons, these tests were constructed as a result of binary logistic function and included two classes of fibrosis stages. For example, they allow the diagnostic of the presence of significant fibrosis, with the classes F0/1 (absence of significant fibrosis) and F2/3/4 (presence of significant fibrosis). Another example is the diagnostic of cirrhosis, with the classes F0/1/2/3 (absence of cirrhosis) and F4 (presence of cirrhosis). The currently most accurate tests present an accuracy of about 75% of correctly classified patients regarding significant fibrosis. However, due to the existing 25% of misclassified patients, a biopsy remains regularly prescribed for patients suspected with severe fibrosis in order to confirm the diagnostic, especially in the indeterminate zone. There is thus a need for an improved non-invasive method leading to a higher diagnostic accuracy and precision (increasing the number of fibrosis classes, with comparison to the binary function, above two), in order to lower the need of liver biopsy.

In order to improve the possibility of distinguishing several fibrosis stages and/or necrotico-inflammatory activity grades, better than with a single binary logistic function, a statistical analysis using discriminant analysis and/or polynomial logistic regression was proposed. This lead to a classification with 5 classes or more, but the classification accuracy was insufficient or even low at about 50% compared to about 75% for a binary diagnosis.

The Inventors described a non-invasive method (hereinafter referred to "2008 RDI Method"), adapted from FibroMeter™ analysis and involving the measure of RDI (Reliable Diagnosis Interval, Cales et al., Liver International, 2008). This method usually combines the diagnostic cut-off of a binary diagnosis with the thresholds of 90 to 95% predictive values of a test, resulting in a classification with four classes, namely F0/1, F1/2, F1/2/3, F2/3/4, and presenting a high accuracy (89.5% of well classified patients). However, these RDIs lead to broad classes, where it is unclear in what extent the patient has to be treated, and the need of biopsy may remain.

Now, the Inventors propose a new invention overcoming this drawback, which is an improved non-invasive method for assessing the presence and/or the severity of lesions, such as, for example, liver fibrosis, based on a new detailed classification and resulting in the ability of ranging or sorting the patient more precisely according to the Metavir stage, and as or more accurately than with the 2008 RDI Method.

### SUMMARY

The present invention thus relates to a non-invasive method for assessing the presence and/or severity of a lesion in an organ of an animal, said method comprising carrying out at least one non-invasive test resulting in a score/value, and positioning the at least one score/value in a class of a detailed classification based on population percentiles, or in a reliable diagnostic interval (RDI), to be crossed with another RDI for a final positioning of both RDIs in a class.

According to an embodiment, the animal is a mammal, such as, for example, a rat or a pet, such as, for example, a cat or a dog. According to a preferred embodiment, the animal is a human.

According to an embodiment, the organ is the liver and the detailed classification is a detailed fibrosis classification and/or a detailed necrotico-inflammatory activity classification.

According to an embodiment, the animal, including a human, is at risk of suffering or is suffering from a condition selected from the group comprising a liver impairment, a chronic liver disease, a hepatitis viral infection especially an infection caused by hepatitis B, C or D virus, an hepatoxicity, a liver cancer, a steatosis, a non-alcoholic fatty liver disease (NAFLD), a non-alcoholic steatohepatitis (NASH), an autoimmune disease, a metabolic liver disease and a disease with secondary involvement of the liver.

According to an embodiment, hepatoxicity is alcohol induced hepatoxicity and/or drug-induced hepatoxicity (i.e. any xenobiotic compound like alcohol or drug).

According to an embodiment, autoimmune disease is selected from the group consisting of autoimmune hepatitis 5 (AIH), primary biliary cirrhosis (PBC) and primary sclerosing cholangitis (PSC).

According to an embodiment, metabolic liver disease is selected from the group consisting of hemochromatosis, Wilson's disease and alpha 1 anti-trypsin deficiency.

According to an embodiment, said disease with a secondary involvement of the liver is celiac disease or amyloidosis.

According to an embodiment, the non-invasive test comprises at least one combination score, obtained by synchronous binary combination of at least one biomarker, at least one clinical marker, at least one data resulting from a physical method and/or at least one score.

Advantageously, said combination score is selected from the group comprising ELF, FIBROSpect™, APRI, FIB-4, Hepascore, Fibrotest™, FibroMeter™ and CirrhoMeter™, preferably said non-invasive score is a FibroMeter^{3G}.

In an embodiment of the invention, said physical method is selected from the group comprising medical imaging data, preferably is selected from the group comprising ultrasonography, especially Doppler-ultrasonography, elastometry ultrasonography and velocimetry ultrasonography, such as, for example, Fibroscan, ARFI, VTE; IRM; and MNR, especially MNR elastometry or velocimetry, more preferably the physical method is Fibroscan™.

According to an embodiment of the invention, the method of the invention comprises carrying out at least one non-invasive test resulting in a score/value, and positioning the at least one score/value in a class of a detailed fibrosis and/or activity classification based on percentiles, wherein said classification is based on the discretization of the scores of a reference population into at least 10 percentiles of 10% of the population, preferably into at least 20 percentiles of 5% of the population, more preferably into 40 percentiles of 2.5% of the population.

In an embodiment, the method of the invention comprises the steps of performing at least two non-invasive tests resulting in at least two scores/values.

Advantageously, said at least two non-invasive tests are FibroMeter™ and Fibroscan™.

According to an embodiment, the non-invasive method of the invention comprises the steps of:
- combining the scores/values obtained from two non-invasive tests in at least two binary logistic regressions to obtain at least two indexes,
- positioning each index on a RDI, wherein the position of RDI has been determined from a reference population
- combining both RDIs according to a double entry table of RDIs showing combined classes. An example of such a double entry table is given below: 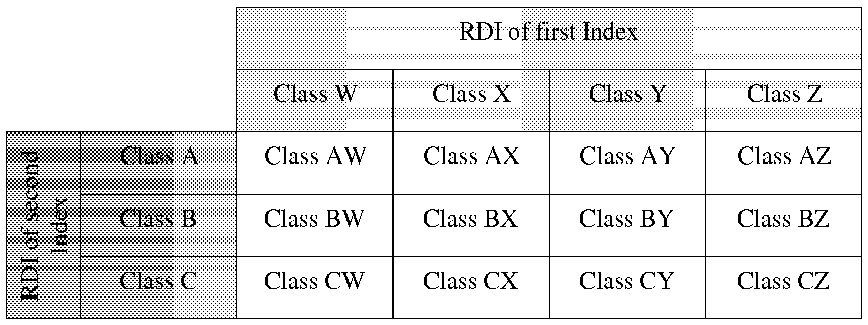
- positioning the RDIs in a combined class (such as, in the above table, classes AW to CZ).

The present invention also refers to a device carrying out the non-invasive method of the invention.

According to an embodiment, the device is a meter reflecting the detailed classification, such as, for example, the new detailed fibrosis stage classification or the new detailed necrotico-inflammatory activity grade classification, based on the discretization of the scores of a reference population into percentiles.

According to another embodiment, the device is a meter reflecting the detailed classification, such as, for example, the new detailed fibrosis stage classification or the new detailed necrotico-inflammatory activity grade classification, based on the combination of reliable diagnostic intervals.

### DEFINITIONS

**About:** Preceding a figure means plus or less 2% of the value of said figure.
**Percentile**: Corresponds to an interval in which a certain percent of observations fall. For example, when dividing a population in 10 percentiles of 10%, each percentile contains 10% of the population.
**Single fibrosis test**: Corresponds to already published blood fibrosis test obtained by a biomarker combination (Fibrotest™, FibroMeter™, Hepascore, APRI or FIB-4, for example), or Fibroscan.
**Combined fibrosis index:** New fibrosis and/or necrotico-inflammatory activity test combining *single fibrosis tests.*
**Reliable diagnosis interval (RDI):** RDIs correspond to the intervals of fibrosis and/or necrotico-inflammatory activity test values wherein the individual diagnostic accuracy is considered sufficiently reliable for clinical practice.
**Youden index:** The index is defined as sensitivity + specificity - 1, where sensitivity and specificity are calculated as proportions. Youden's index has minimum and maximum values of -1 and +1, respectively, with a value of +1 representing the optimal value for an algorithm.
**Score/value:** Correspond to the result of a non-invasive test, wherein the result is a number. In the present invention, a score specifically means a result of a non-invasive test ranging from 0 to 1 as obtained by the logic function uses in the binary logistic regression.

### DETAILED DESCRIPTION

The present invention relates to a non-invasive method for assessing the presence and/or severity of a lesion in an organ based on a new detailed stage classification. According to an embodiment, the organ is liver, and the detailed stage classification is a detailed fibrosis classification and/or a detailed necrotico-inflammatory activity classification. According to an embodiment, the method of the invention is useful for assessing the presence and/or severity of liver fibrosis or cirrhosis.

The method of the invention implements a non-invasive test or method of the prior art, such as for example a score based on the measure of biomarkers and/or clinical markers, and/or physical data such as for example medical imaging data. However, the method of the invention differs from the prior art by the classification underlying the test.

### [Scores]

According to an embodiment, the non-invasive test comprises the measure of at least one combination score, obtained by synchronous binary combination of at least one biomarker, at least one clinical marker, at least one data resulting from a physical method and/or at least one score. Scores that may be used for assessing presence or severity of a disease, such as, for example, a liver disease, are well known in the art. Examples of scores include, but are not limited to ELF, FIBROSpect™, APRI, FIB-4, Hepascore, Fibrotest™, FibroMeter™ and CirrhoMeter™.

ELF is a blood test based on hyaluronic acid, P3P, TIMP-1 and age. FIBROSpect™ is a blood test based on hyaluronic acid, TIMP-1 and A2M. APRI is a blood test based on platelet and AST.

FIB-4 is a blood test based on platelet, ASAT, ALT and age.

HEPASCORE is a blood test based on hyaluronic acid, bilirubin, alpha2-macroglobulin, GGT, age and sex.

FIBROTEST™ is a blood test based on alpha2-macroglobulin, haptoglobin, apolipoprotein A1, total bilirubin, GGT, age and sex.

FIBROMETER™ is a family of blood tests, the content of which depends on the cause of chronic liver disease and the diagnostic target with details in the following table:

| FibroMeter | Age | Sex | A2M | AH | PI | PLT | AST | Urea | GGT | Bili | ALT | Fer | Glu |
|---|---|---|---|---|---|---|---|---|---|---|---|---|---|
| F virus | X | X | X | X | X | X | X | X | | | | | |
| AOF virus | | | X | X | | X | | X | X | X | | | |
| F alcohol | X | | X | X | X | | | | | | | | |
| AOF alcohol | | | X | X | X | X | | | | | | | |
| F NAFLD | X | | | | | X | X | | X | X | X | X | X |
| AOF NAFLD | | | | X | X | X | X | | | | X | | X |

A2M: alpha2-macroglobulin, HA: hyaluronic acid, PI: prothrombin index, PLT: platelets, Bili: bilirubin, Fer: ferritin, Glu: glucose, F: fibrosis stage (Metavir), AOF: area of fibrosis, NAFLD: non-alcoholic fatty liver disease

According to an embodiment, said score is a FibroMeter™, preferably a FibroMeter™ of third generation (FibroMeter^{3G})

| | | | | | | | | |
|---|---|---|---|---|---|---|---|---|
| FibroMeter^{3G} | Age | Sex | A2M | PI | PLT | AST | Urea | GGT |
| | X | X | X | X | X | X | X | X |

### [Biomarkers and clinical markers]

According to an embodiment, the at least one biomarker is selected from the group comprising Glycemia, AST (aspartate aminotransferase), ALT (alanine aminotransferase), AST/ALT, AST.ALT, Ferritin, Platelets (PLT), Prothrombin time (PT), Hyaluronic acid (HA or hyaluronate), Haemoglobin, Triglycerides, Alpha-2 macroglobulin (A2M), Platelets, Gamma-glutamyl transpeptidase (GGT), Prothrombin index (PI), Urea, Bilirubin, apolipoprotein A1 (ApoA1), type III procollagen N-terminal propeptide (P3P), gamma-globulins (GLB), sodium (NA), albumin (ALB), alkaline phosphatases (ALP), YKL-40 (human cartilage glycoprotein 39), tissue inhibitor of matrix metalloproteinase 1 (TIMP-1), cytokeratine 18 and matrix metalloproteinase 2 (MMP-2) to 9 (MMP-9).

According to an embodiment, the at least one clinical marker is selected from the group comprising weight, body mass index, age, sex, hip perimeter, abdominal perimeter and the ratio thereof, such as for example hip perimeter/abdominal perimeter.

### [Physical data]

According to an embodiment, the non-invasive test comprises the measure of at least one data issued from a physical method of diagnosing liver fibrosis.

According to an embodiment, said physical method is selected from the group comprising medical imaging data.

According to an embodiment, the physical method is selected from the group comprising ultrasonography, especially Doppler-ultrasonography and elastometry ultrasonography and velocimetry ultrasonography (Fibroscan, ARFI, VTE), IRM, and MNR, especially MNR elastometry or velocimetry. Preferably, the data are Liver Stiffness Evaluation (LSE) data. According to a preferred embodiment of the invention, the data are issued from a Fibroscan.

### [Classifications which are underlying the method of the invention]

According to the invention, the new detailed fibrosis stage and/or necrotico-inflammatory activity grade classification results from the statistical analysis of the data obtained from at least one non-invasive test as above-described, in a reference population of patients with chronic liver disease.

### Reference population

According to an embodiment, in order to set up the new detailed fibrosis stage and/or necrotico-inflammatory activity grade classification, a reference population of patients with chronic liver disease is required. According to an embodiment, the reference population may be a population of patients affected with a Hepatitis virus, preferably with the Hepatitis C virus. According to an embodiment, the reference population contains at least about 500 patients, preferably at least about 700 patients, more preferably at least about 1000 patients.

According to an embodiment, in order to set up the new detailed fibrosis stage and/or necrotico-inflammatory activity grade classification, the following data are needed for each patient of the reference population:
- at least one non-invasive score or imaging data as here-above described, and
- a histological staging, preferably a histological staging according to the Metavir system, obtained by a liver biopsy.

The classifications underlying the method of the invention are selected from the group consisting of percentiles and RDI combination.

### Percentiles

According to an embodiment, the new detailed fibrosis stage classification or the new detailed necrotico-inflammatory activity grade classification is based on the discretization of the results of the non-invasive test into percentiles. According to a preferred embodiment, the result of the non-invasive test is a score, preferably a FibroMeter™ score.

Advantageously, the percentiles are percentiles of patients, i.e. each percentile contains the same number of patients. In an embodiment, the percentiles are not percentiles of values obtained with the non-invasive test.

According to an embodiment, the population of patients is classified into at least 10 percentiles of 10% of the population, preferably into at least 20 percentiles of 5% of the population, more preferably into 40 percentiles of 2.5% of the population.

According to an embodiment, for each percentile, the number of patients diagnosed after a liver biopsy at each Metavir F stage (F0 to F4) and/or at each Metavir A grade (A0 to A3) is quantified. Advantageously, a table is drawn, with lines corresponding to percentiles and column corresponding to histological Metavir F stage and/or A grade classification. For example, in the **Table 1** below, each line represents a percentile (here is shown a classification in 10 percentiles of 10%) and each column represents a Metavir F stage. Each x_{i,j} represents the number of patients of percentile i in Metavir F stage j.

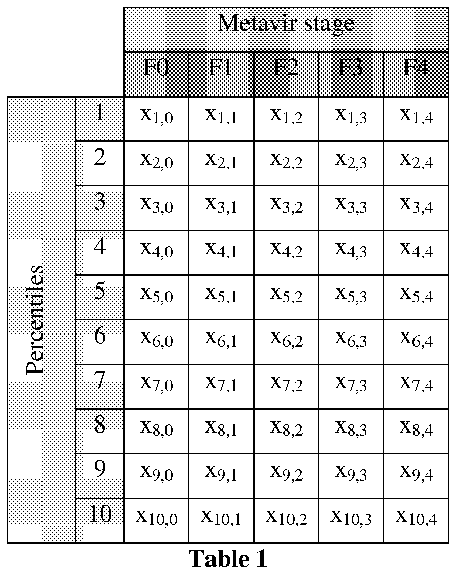

First, for each percentile (i.e. for each line of the table drawn as described here-above), the most frequent histological Metavir F stage and/or A grade is determined. For example, in table 1, the highest x_{i,j} of each line is determined.

Second, the minimal correct classification rate is fixed per percentile at more than about 75%, preferably of more than about 80%, more preferably of more than about 85%, even more preferably of more than about 90%. On each line, the box containing the highest number is selected; further selected is the contiguous box on same line having preferably the second highest number which, when summed with the previous highest number, is equal or higher than the above-mentioned rate. When this situation does not occur, a third contiguous box having preferably the third highest number is selected, in order to equal the above-mentioned rate. Preferably, the contiguous box is selected towards to higher Metavir F stage and/or A grade.

For example, in **Table 2** below, on each line, the highest value is in bold and the selected boxes are represented in grey.

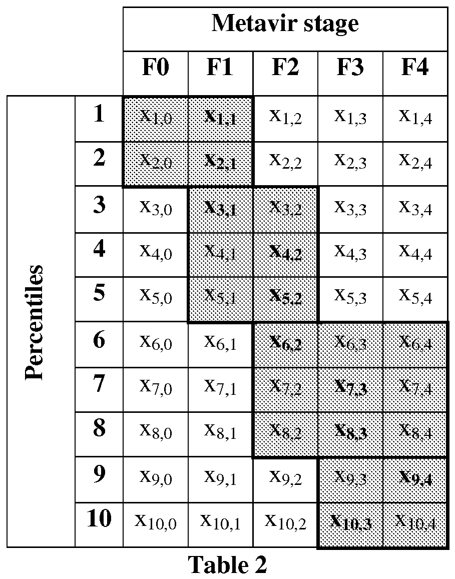

When the selected columns are the same on two contiguous lines, both lines are grouped. For example, in table two, the lines of Percentiles 1 and 2 are grouped.

All the selected boxes of a group of lines form a block. For example, in **Table 2,** x_{1,0}, x_{1,1}, x_{2,0} and x_{2,1} for a group.

Each block corresponds to a class. For example, the block formed by x_{1,0}, x_{1,1}, x_{2,0} and x_{2,1} corresponds to the F0/F1 class. In the example of **Tables 1** and **2**, the classification thus comprises 4 classes (F0/1, F1/2, F2/3/4 and F3/4).

The limits of the first class are determined by the lowest and highest score values obtained by a patient of said class. The upper limit of the following classes is determined by the highest score value obtained by a patient of each class. For example, in **Table 2**, the highest value of class F1/2 corresponds to the highest value obtained to the non-invasive test by a patient of the class F1/2 (i.e. a patient of x_{3,1}, x_{3,2}, x_{4,1}, x_{4,2}, x_{5,1} and x_{5,2}).

In an embodiment, the reference population is a population of patients affected with Hepatitis C Virus, the non-invasive test is a FibroMeter™ and the population is segmented in 40 percentiles of 2.5%.

In another embodiment, the reference population is a population of patients affected with Hepatitis C Virus, the non-invasive test is a FibroMeter™ and the population is segmented in 20 percentiles of 5%.

According to an embodiment, the classification based on percentiles as here-above described comprises at least 3 classes, preferably at least 4 classes, more preferably at least 5 classes, even more preferably at least 6 classes, even more preferably at least 7 classes.

According to an embodiment, the classification based on percentiles comprises 7 classes, namely F0/1, F1, F1/2, F1/2/3, F2/3, F2/3/4 and F3/4. According to this embodiment, the classification was implemented from a reference population to which a score based on a binary regression logistic function was performed, preferably FibroMeter™. The threshold value of each class is indicated in the second line of the table below:

| **F0/1** | | **F1** | | **F1/2** | | **F1/2/3** | | **F2/3** | | **F2/3/4** | | **F3/4** | |
|---|---|---|---|---|---|---|---|---|---|---|---|---|---|
| 0 | 0.10-0.15 | | 0.15-0.20 | | 0.20-0.65 | | 0.65-0.80 | | 0.80-0.95 | | 0.95-1 | | 1 |
| | pref 0.14 | | pref 0.17 | | pref 0.56 | | pref 0.72 | | pref 0.86 | | pref 0.97 | | |

| | | | | | | | | | | | | | |
|---|---|---|---|---|---|---|---|---|---|---|---|---|---|
| Pref: preferably | | | | | | | | | | | | | |

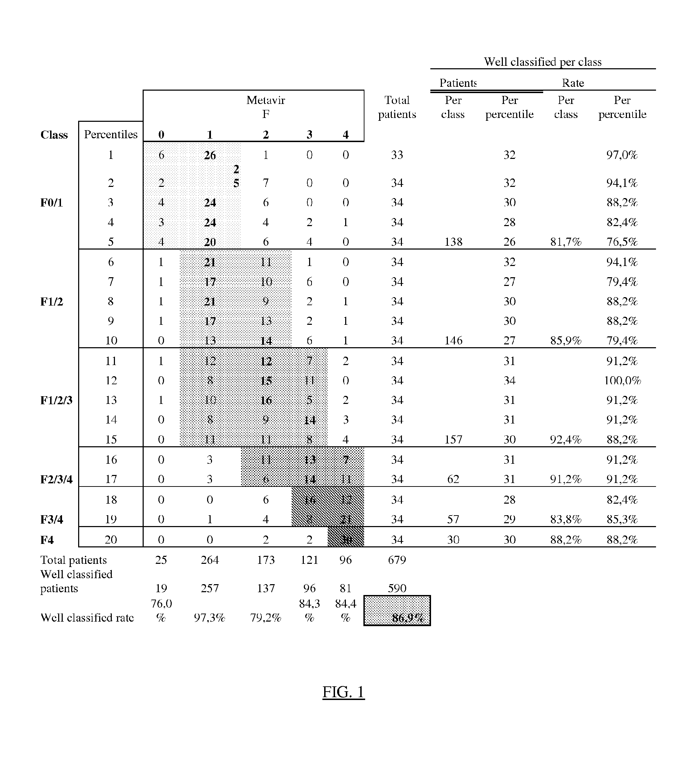

An example of a classification based on percentiles as here-above described is shown in **Figure 1**.

### [Meter]

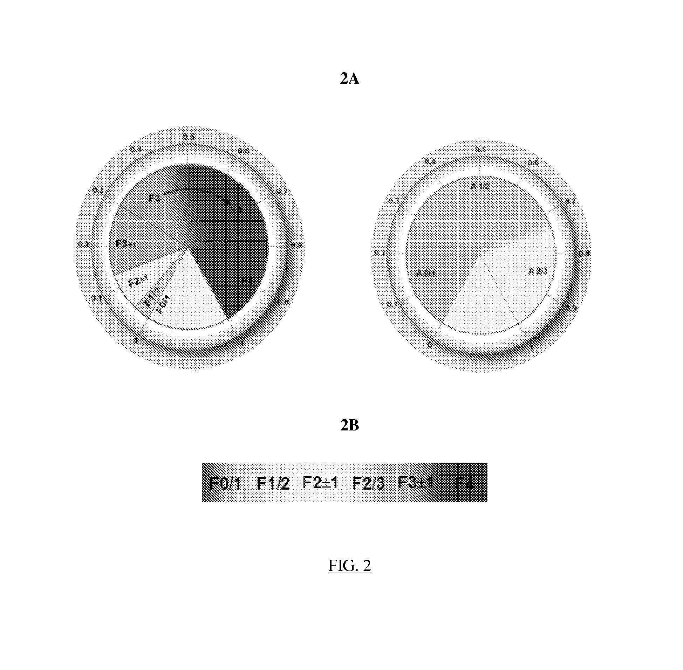

Another object of the invention is a device carrying out the method of the invention. Preferably, the device is a meter, reflecting the detailed classification, such as, for example, the new detailed fibrosis stage and/or necrotico-inflammatory activity grade classification based on percentiles as here-above described. Examples of meters are represented in **Figure 2A** for a classification based on a FibroMeter™ score, referring to fibrosis Metavir stages (F) and to necrotico-inflammatory activity Metavir grades (A).

According to an embodiment, the meter indicates the sectors corresponding to the blocks of the classification, and the corresponding stage of fibrosis and/or grade of necrotico-inflammatory activity. The meter comprises scale marks corresponding to the score. According to an embodiment, said scale marks range from 0 to 1. The sectors are sequentially positioned on the meter. According to an embodiment, each sector of the meter has a different color.

According to a first embodiment, said meter is in the form of a line. According to a second embodiment, said meter is in the form of a disk.

According to an embodiment, the meter comprises a means, for example a line or an arrow, indicating the score obtained by one patient to the non-invasive test and ranging the patient in a class. This indicator allows a direct visualization of the method of the invention.

### [Associated method]

An object of this invention is thus a non-invasive method implementing the above-described classification.

In a first step of the method, a non-invasive test is carried out in a patient and gives a result in the form of a value, preferably of a score. According to the invention, said non-invasive test is the same as the one used in the classification.

In a second step, said value, preferably said score, is positioned on said classification, optionally on said meter, in order to range the patient in a given class.

The accuracy of the method of the invention (i.e. the rate of well classified patients) is of at least about 75%, preferably of at least about 80%, more preferably of at least about 85%, even more preferably of at least about 90%. According to the invention, this accuracy depends on the minimal correct classification rate as described in the second step.

### RDI

### [Measure of RDI]

According to an embodiment, the new detailed fibrosis stage and/or necrotico-inflammatory activity grade classification is based on the measure of Reliable Diagnosis Intervals (RDIs) obtained from scores/values of a reference population. According to a preferred embodiment, the scores/values are obtained from the group comprising Fibroscan™, Fibrotest, FibroMeter™, CirrhoMeter™, Hepascore, FIB-4 and APRI.

By nature, RDIs are specific for a diagnostic target. According to a first embodiment, the diagnostic target is clinically significant fibrosis (CSF), i.e. Metavir F≥2. According to a second embodiment, the diagnostic target is severe fibrosis (SF), i.e. Metavir F≥3. According to a third embodiment, the diagnostic target is cirrhosis (C), i.e. Metavir F=4.

RDI measurement is well known in the art. Briefly, said measurement is based on the division of the scores/values obtained in a reference population (as here-above described) into several consecutive intervals.

According to an embodiment, the measurement of the RDI comprises two, and optionally three steps:
- in a first step, negative (NPV) and positive (PPV) predictive values are calculated. The method for calculating NPV and PPV from a population is well known in the art. In order to calculate these values, a threshold is arbitrarily fixed. According to an embodiment, the threshold is equal to about 75%, preferably to about 80%, about 85%, about 90%, about 95%, and more preferably to about 98%;
- in a second step, using NPV and PPV, two intervals are defined among the scores/values: (i) a lower interval, defined by a score/value inferior or equal to NPV value (score threshold), wherein patients have more than 90% chances of not entering into the diagnostic target; and (ii) a higher interval, defined by a score/value superior or equal to PPV value (score threshold), wherein patients present a risk superior to 90% of entering into the diagnostic target; and
- optionally, in a third step, the remaining intermediate interval (scores/values between NPV and PPV score threshold) is segmented in two supplemental intervals according to the fibrosis and/or necrotico-inflammatory activity test value providing the diagnostic cut-off of a binary diagnosis for the diagnostic target like the maximum Youden index or the maximum diagnostic accuracy. By nature, these two intervals correspond to a class of fibrosis stages different from the initial diagnostic target but with a combined prevalence of fibrosis stages and/or necrotico-inflammatory activity grades providing a class accuracy superior or equal to the predetermined threshold of fibrosis stage prevalence (e.g. ≥ 75%).

Therefore, for each diagnostic target, three or four intervals are defined. Each interval corresponds to a class of fibrosis stage(s) and/or necrotico-inflammatory activity grade(s). A given patient may be ranged in one of these intervals according to the score/value obtained by said patient to the non-invasive test.

### [New combined fibrosis indexes]

According to an embodiment, in order to improve the diagnostic accuracy of the method of the invention based on the RDI method as here-above described, *Single fibrosis and*/*or necrotico-inflammatory activity tests* are combined and *New combined fibrosis and*/*or necrotico-inflammatory activity indexes* are obtained. According to an embodiment, *Single fibrosis and*/*or necrotico-inflammatory activity tests* are selected from the group comprising Fibroscan™, Fibrotest, FibroMeter™, CirrhoMeter™, Hepascore, FIB-4 and APRI.

To identify the best combination of *single fibrosis and*/*or necrotico-inflammatory activity tests* for the assessment of the presence of significant fibrosis, a stepwise binary logistic regression is performed and repeated on about 500, preferably on about 750, more preferably on about 1,000 bootstrap samples in an exploratory set of patients. The bootstrap method consists of a repeated sampling (with replacement) from the original entire dataset, followed by a *stepwise* logistic regression procedure in each subsample. The most frequently (>50%) selected *single fibrosis andlor necrotico-inflammatory activity tests* among the about 500, preferably on about 750, more preferably on about 1,000 analyses are then included in a single binary logistic regression performed in the whole population of the exploratory set.

According to an embodiment, using the regression score of such a multivariate analysis, new *combined fibrosis andlor necrotico-inflammatory activity indexes* are constructed for each diagnostic target, ranging from 0 to 1. According to an embodiment, for clinically significant fibrosis, said index is called "CSF-index". According to another embodiment, a "SF-index" is constructed for the assessment of the presence of severe fibrosis as well as a "C-index" for the assessment of the presence of cirrhosis, according to methods well-known from the skilled artisan.

According to an embodiment, the combined fibrosis index used in the present invention is based on the combination of FibroMeter™ and FibroScan™.

According to an embodiment, RDIs are calculated for each of the combined fibrosis index (CSF-index, SF-index and C-index). As described here above, 3 or 4 RDIs are obtained for each index.

According to an embodiment, said indexes are based on the combination of FibroMeter™ and Fibroscan™ scores/values, and range between 0 and 1.

According to an embodiment, for the SCF-index, 4 intervals are defined, corresponding to the following stage of fibrosis classes: F0/1, F1/2, F1/2/3, F2/3/4. The intervals correspond to the following values of the SCF-index:
- F0/1 : from 0 to about 0.2 to 0.3, preferably to about 0.235;
- F1/2: from 0.2 to 0.3, preferably from about 0.235; to about 0.35 to 0.45, preferably to about 0.415;
- F1/2/3: from 0.35 to 0.45, preferably from about 0.415; to about 0.55 to 0.75, preferably to about 0.636;
- F2/3/4: from about 0.55 to 0.75, preferably from about 0.636, to 1.

According to an embodiment, for the SF-index, 4 intervals are defined, corresponding to the following stages of fibrosis classes: F0/1/2, F1/2/3, F2/3/4, F3/4. The intervals correspond to the following values of the SF-index:
- F0/1/2: from 0 to about 0.15 to 0.30, preferably to about 0.220;
- F1/2/3: from 0.15 to 0.30, preferably from about 0.220; to about 0.30 to 0.45, preferably to about 0.364;
- F2/3/4: from 0.30 to 0.45, preferably from about 0.364; to about 0.70 to 0.95, preferably to about 0.870;
- F3/4: from about 0.70 to 0.95, preferably from about 0.870, to 1.

According to an embodiment, for the C-index, 3 intervals are defined, corresponding to the following stages of fibrosis classes: F0/1/2/3, F2/3/4, F4. The intervals correspond to the following values of the SF-index:
- F0/1/2/3 : from 0 to about 0.15 to 0.35, preferably to about 0.244;
- F2/3/4: from 0.15 to 0.35, preferably from about 0.244; to about 0.60 to 0.95, preferably to about 0.896;
- F4: from about 0.60 to 0.95, preferably from about 0.896, to 1.

According to an embodiment, the RDIs obtained for each index are combined, in order to obtain a more detailed classification. According to a first embodiment, the RDIs of CSF-index and of SF-index are combined, leading to the "CSF/SF classification". According to a first embodiment, the RDIs of CSF-index and of C-index are combined, leading to the "CSF/C classification".

According to an embodiment, said combination corresponds to the drawing of a double entry table, comprising columns corresponding to RDIs of the first index, and lines corresponding to RDIs of the second index. An example of such a double entry table based on Metavir F stages, is **Table 3** below.

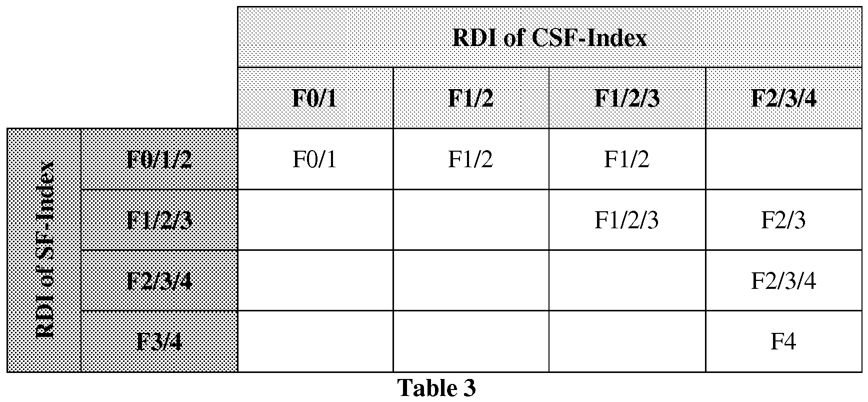

For each patient, the calculated CSF-Index is positioned in one of the RDIs of CSF-index, and the calculated SF-Index is positioned in one of the RDI of SF-Index. As an example, a patient with a CSF-Index positioned in the class F2/3/4 and with a SF-Index positioned in the class F1/2/3 may be classified in a narrower class F2/3.

Therefore, as shown in **Table 3**, the combination of RDIs or CSF-index and of SF-index leads to a "CSF/SF classification", comprising 6 classes, namely F0/1, F1/2, F1/2/3, F2/3, F2/3/4 and F4.

Accordingly, and as illustrated on **Table 4** below, the combination of RDIs or CSF-index and of CF-index leads to a "SCF/CF classification" comprising 7 classes, namely F0/1, F1/2, F1/2/3, F2, F2/3, F2/3/4 and F4.

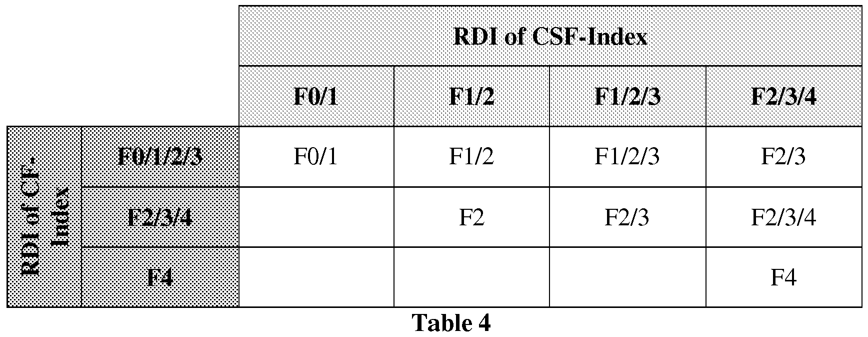

According to an embodiment, said new fibrosis stage and/or necrotico-inflammatory activity grade classification comprises at least 3, preferably at least 4, more preferably at least 5 classes, even more preferably at least 6 or 7 classes.

### [Meter]

Another object of the invention is a device carrying out the method of the invention. Preferably, the device is a meter, reflecting the detailed classification, such as, for example, the new detailed fibrosis stage and/or necrotico-inflammatory activity grade classification as here-above described. Examples of meters are represented in **Figure 2B** for a fibrosis classification based on the combination of FibroMeter™ and FibroScan™.

According to a first embodiment, said meter is in the form of a line. According to a second embodiment, said meter is in the form of a disk.

According to an embodiment, the Meter of the invention is segmented in different sectors, each sector corresponding to a class of the classification. According to an embodiment, each sector of the meter has a different color.

### [Associated non-invasive method]

An object of this invention is thus a non-invasive method implementing the new detailed fibrosis stage and/or necrotico-inflammatory activity grade classification as here-above described.

In a first step of the method, at least two non-invasive tests are carried out in a patient and at least two values/scores are obtained. According to the invention, said non-invasive tests are selected from the group comprising Fibroscan™, Fibrotest, FibroMeter™, CirrhoMeter™, Hepascore, FIB-4 and APRI, and are the same as the ones used to obtain the classification. Preferably, said non-invasive tests are Fibroscan™ and FibroMeter™.

In a second step, both results are combined using three binary logistic regressions to obtain three indexes (CSF-Index, SF-Index and C-Index), ranging from 0 to 1.

In a third step, the CSF-Index SF-Index and C-Index are ranged respectively in a RDI of CSF-Index, in a RDI of SF-Index and in a RDI of C-Index.

In a fourth step, using a double entry table (see Tables 3 and 4), the patient is positioned in a fibrosis stage and/or necrotico-inflammatory activity grade class.

According to an embodiment, the accuracy of said non-invasive method (i.e. the rate of well classified patients) is of at least about 75%, preferably of at least about 80%, more preferably of at least about 85%, even more preferably of at least about 90%.

### [Advantages]

The non-invasive methods of the present invention, implementing new detailed fibrosis stage and/or necrotico-inflammatory activity grade classifications based on percentiles or on the combination of RDIs, both present the following advantages:
- Increased precision, due to the number of classes of the classification,
- Statistically significant increase in diagnostic accuracy, with an accuracy > 75%,
- The possibility to target this classification towards different diagnostic targets,
- The possibility to apply this classification to different non-invasive tests or methods, especially by combining two or more non-invasive tests.

### BRIEF DESCRIPTION OF THE DRAWINGS

**Figure 1****: Example of classification based on percentiles.**
**Figure 2****: Examples of Meters of the invention. (A) Meter reflecting the detailed** classification based on percentiles, referring to fibrosis (F) and to necrotico-inflammatory activity (A); (B) Meter reflecting the detailed classification based on the combination of RDIs.
**Figure 3****: Various fibrosis stage classifications. A:** Histological Metavir fibrosis stages. **B-E:** Fibrosis stage classifications by non invasive tests; **B:** Fibrotest classification; **C:** FibroMeter classification; **D:** Fibroscan classification; **E:** new CSF/SF classification derived from the association of new fibrosis indexes combining FibroMeter and Fibroscan.
**Figure 4****: Study methodology.** Implementation of *new fibrosis stage classifications* from new *combined fibrosis indexes* (exploratory set). BLR: binary logistic regression, RDI: reliable diagnosis intervals.

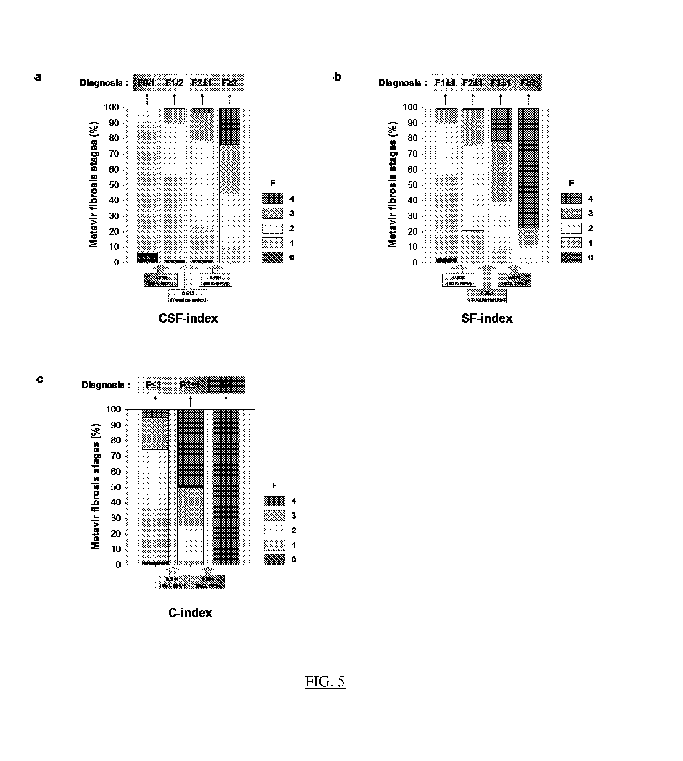

**Figure 5****: Reliable diagnosis intervals of CSF-, SF- and C-indexes in the exploratory set. *Panel s3a:*** Proportion of Metavir fibrosis (F) stages according to the maximum Youden index cut-off and the thresholds of 90% negative and positive predictive values for significant fibrosis with CSF-index. ***Panel s3b:*** Proportion of Metavir F stages according to the maximum Youden index cut-off and the thresholds of 90% negative and positive predictive values for severe fibrosis with SF-index. ***Panel s3c**:* Proportion of Metavir F stages according to the thresholds of 95% predictive values for cirrhosis with C-index.
**Figure 6****: Proportion of Metavir fibrosis (F) stages as a function of CSF/SF classification (X axis with the rate of patients included in each class in italics), in the exploratory (panel 1a) and validation (panel 1b) sets. The bottom line indicates the** *fibrosis stage classification.*
**Figure 7****: Rates of correctly classified patients by *fibrosis stage classifications* as a function of Metavir fibrosis stages in the validation set.** Hatched lines: *single fibrosis tests,* continuous lines: new *fibrosis stage classifications* derived from new *combined fibrosis indexes.* Because of the few number of F0 patients, F0 and F1 were pooled together.
**Figure 8**: Rate of correctly classified patients *by fibrosis stage classifications* as a function of IQR/median ratio in the validation set. IQR is the interquatile range (values from 25 to 75% of patients).

### EXAMPLES

### Example 1: Construction of the classification based on the percentiles

In a population of 1000 patients with chronic liver disease, a FibroMeter was carried out (resulting in a score of fibrosis, ranging from 0 to 1) as well as a biopsy, resulting in a histological staging using the Metavir system, ranging from F0 to F4.

The population is discretized in 40 percentiles of 2.5% according to the score of fibrosis. The **Table 5** is drawn, wherein the classes of histological reference are in columns and the previous percentile classes in lines.

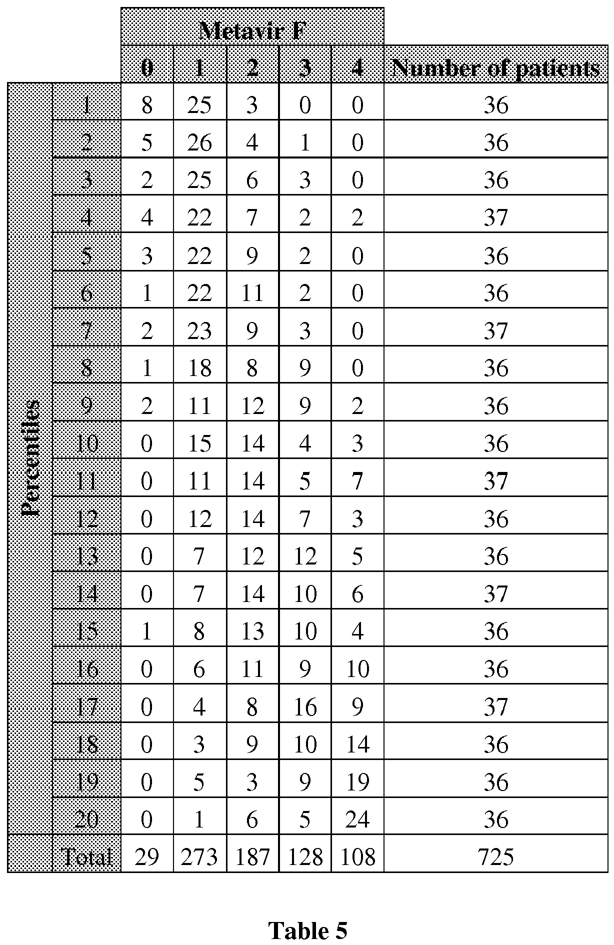

The most frequent histological stage in each percentile class is determined. In the following example, the most frequent stages per percentile are indicated in bold characters **(Table 6).**

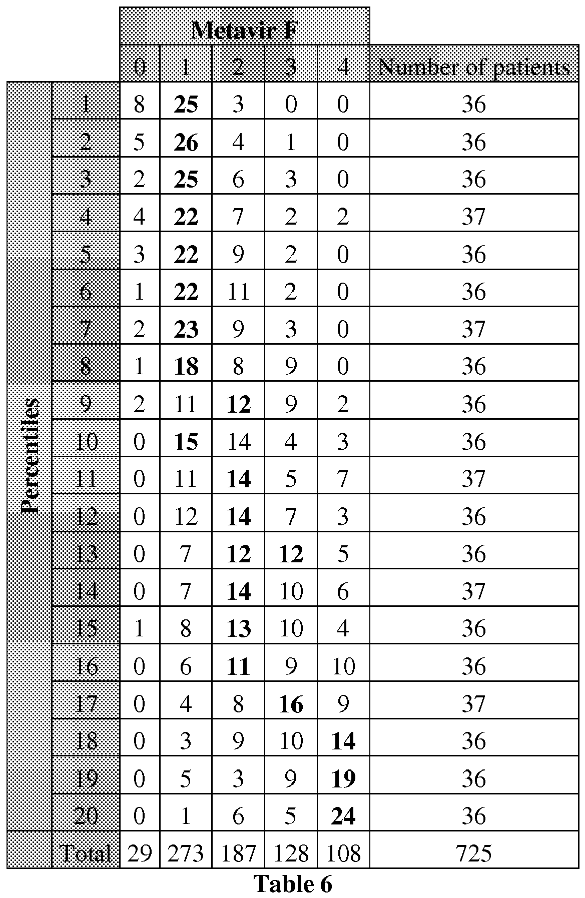

The rate of well classified patients in each line is then calculated.

On the first percentile line, the minimum number of contiguous columns (histological fibrosis stages) in order to reach a predefined minimum correct classification rate ≥ 80% is selected. Then, this process is expended to the next line until the correct classification rate is ≥ 80%.

When this correct classification rate declines, especially when it is lower than the predefined rate, one or contiguous column(s) with higher fibrosis stages (to the right hand of the table) are further selected to reach again the predefined minimum correct classification rate. Then, the correct classification rate for each histological stage on a bottom line is calculated (**Table 7**). It should be noted that the prevalence of F0 stage in usually low in this kind of study due to the low prevalence of liver biopsy in this stage. Therefore, the second preferred stage in the first class is F0 by convention to circumvent this bias.

Five fibrosis classes (this is an example): F0/1, F1/2, F1/2/3, F2/3/4, F3/4 (in grey on Table 7) are obtained, with an overall accuracy of 85.8%.

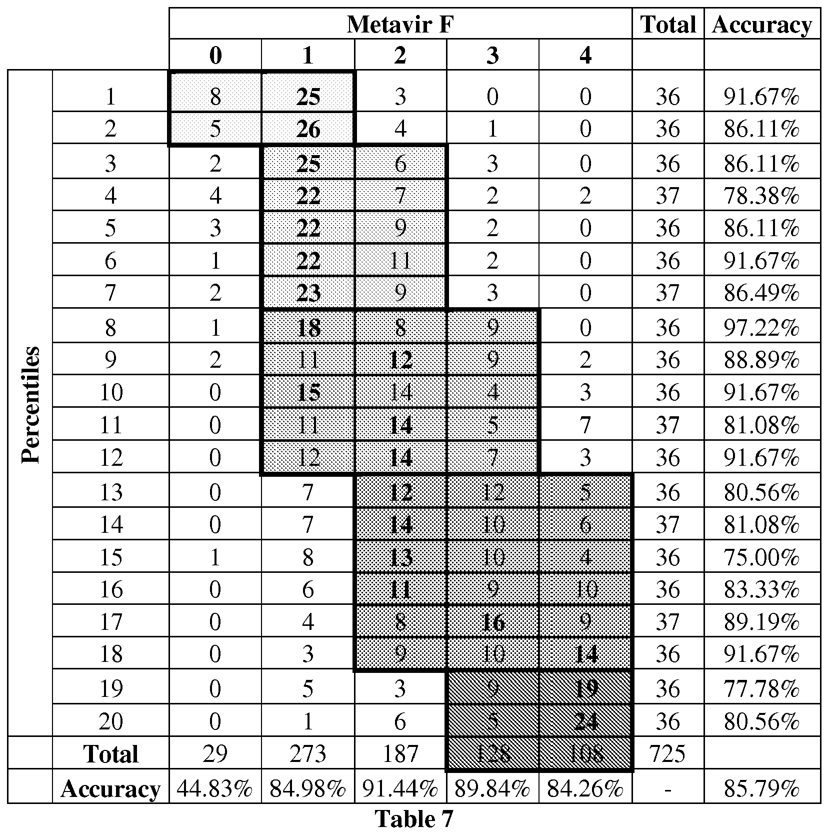

The thresholds of each fibrosis class are obtained from the data base (Table 8).

**Table 8**

| **Equivalent Metavir F** | **Score of fibrosis (FibroMeter)** |
|---|---|
| | 0 |
| F0/1 | |
| | 0,13925337 |
| F1 | |
| | 0,17132949 |
| F1/2 | |
| | 0,55542014 |
| F1/2/3 | |
| | 0,72255544 |
| F2/3 | |
| | 0,86852787 |
| F2/3/4 | |
| | 0,97262959 |
| F3/4 | |
| | 1 |

### Example 2: Classification based on the percentiles

### METHODS

### Study design

We recruited different populations with liver biopsy to evaluate the different diagnostic means. Thus, populations #1, #2 and #3 included blood tests. The three populations were separately analysed due to their initial different designs and to evaluate the accuracy robustness given these differences.

### Populations

Patients with chronic HCV hepatitis, liver biopsy, blood tests and available Fibroscan were consecutively recruited in different populations #1 to #3 described in **Table 9.**

**Table 9: Main characteristics of HCV populations.**

| **Population #** | **Study name** | **Patients (n)** | **Liver biopsy length (mm)** | **Blood FS Metavir F prevalence (%) tests** | | | | | | |
|---|---|---|---|---|---|---|---|---|---|---|
| | | | | | | 0 | 1 | 2 | 3 | 4 |
| 1 | Sniff 17 | 1056 | 21±8 | x | - | 4.4 | 43.5 | 27.0 | 14.0 | 11.2 |
| 2 | Fibrostar | 458 | 25±8 | x | x | 6.7 | 45.1 | 17.9 | 15.6 | 14.8 |
| 3 | Vindiag 7 | 349 | 25±9 | x | x | 1.4 | 30.7 | 35.5 | 20.6 | 11.7 |

| | | | | | | | | | | |
|---|---|---|---|---|---|---|---|---|---|---|
| x: test performed, FS: Fibroscan | | | | | | | | | | |

Each population had different characteristics and fibrosis assessments. Inclusion and exclusion criteria are detailed in previous publications or below for new populations. Briefly, patients did not receive antiviral or known anti-fibrotic treatments. Liver biopsy, blood withdrawal and Fibroscan, when available, were performed within a maximum of 6 months time interval.

*Population #1* included 1056 patients provided by five centers participating in the Sniff 17 study (Cales P et al., Liver Int 2008; 28:1352-62). Thus, individual patient data were available from five centers, independent for study design, patient recruitment, blood marker determination and interpretation of liver histology by an expert pathologist. Blood and pathological determinations were not centralized.

*Population* #2 included 458 patients provided by 19 centers participating in the Fibrostar study (Zarski JP et al., Hepatology 2009; 50:1061A). Blood determination and liver interpretation were centralized. Liver specimens were read by two senior experts, one of whom was from the Metavir group.

*Population #3* included 349 patients provided by three centers participating in the Vindiag 7 study (Boursier J et al., J Hepatol 2010; 52:S405). Blood and pathological (one senior expert in each center) determinations were not centralized.

### Diagnostic means

Fibrosis was staged in liver biopsy according to Metavir staging (The French METAVIR Cooperative Study Group, Hepatology 1994; 20:15-20) in all patients. This fibrosis stage classification was used as the reference for the calculation of accuracy. Blood tests were determined in all studies. We only evaluated here FibroMeter™ (Cales P et al., Hepatology 2005; 42:1373-81, Biolivescale, Angers, France).

### Fibrosis classifications

We distinguished as fibrosis degrees the histological *fibrosis stages* and the *fibrosis classes* provided by non-invasive tests and including one or several fibrosis stages. Several fibrosis classifications were evaluated:
- The histological *fibrosis stage classification* into 5 F_{M} stages, as determined on a liver specimen by a pathologist. This was the reference for accuracy.
- The *binary diagnosis of significant fibrosis* (2 classes) determined either on liver specimen or by the diagnostic cut-off in non-invasive tests. This is the usual diagnostic target of non-invasive tests and thus served as comparator for the detailed classifications. Indeed, as it was expected that a more detailed classification would result in decreased accuracy, this binary accuracy allowed to evaluate this putative accuracy loss.
- The *fibrosis class classification* corresponding to the classification based on percentiles described in the present invention.

### RESULTS

FibroMeter^{3G} shows a significant increase in correct classification rate of *fibrosis class classification* compared to significant fibrosis diagnosis.

### Population #1

### Classification accuracy

The accuracy of *fibrosis class classification* by FibroMeter^{3G} was 86.9% vs. 77.9% for binary diagnosis of significant fibrosis (11.6% relative increase) **(Table 10).**

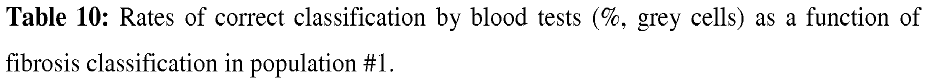

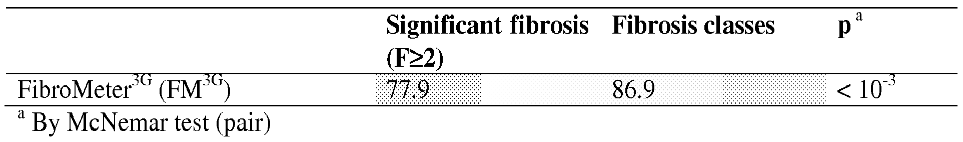

### Populations #2 and 3

In population #2 (and #3), the accuracy of the *fibrosis class classifications* was 77.1% (83.4%) for FibroMeter^{3G} (**Table 11**).

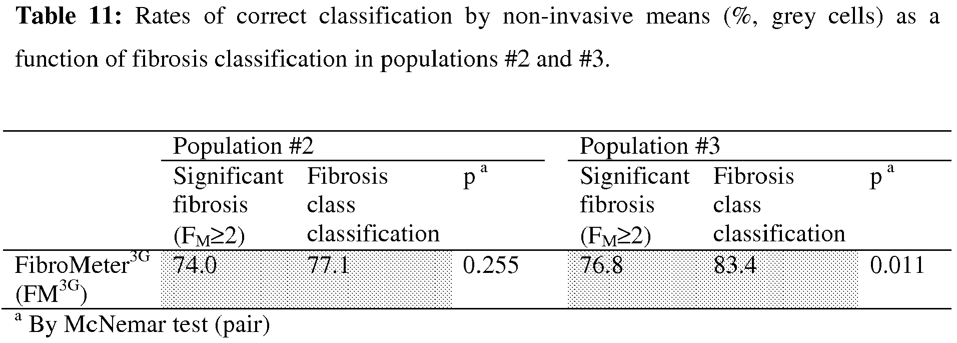

### Example 3: Classification based on the combination of RDIs

### METHODS

### Patients

***Exploratory set** -* Patients with CHC hospitalized for a percutaneous liver biopsy were prospectively enrolled from March 2004 to September 2008 in 3 tertiary centers in France (Angers, Bordeaux, and Grenoble). Patients with cirrhosis complications (ascites, variceal bleeding, systemic infection, hepatocellular carcinoma) were not included. Blood fibrosis tests and Fibroscan were performed in the week preceding biopsy. All patients gave their informed consent. The study protocol conformed to the ethical guidelines of the current Declaration of Helsinki and received approval from the local Ethics committee. ***Validation set** -* The validation set corresponded to the multicenter population of the FIBROSTAR study promoted by the French National Agency for research in AIDS and hepatitis (Zarski JP. et al., J Hepatol 2010; 52:S175). This study prospectively included 512 patients with CHC. All patients had liver biopsy, blood fibrosis tests and Fibroscan. Patients included in both the exploratory set and the FIBROSTAR study were excluded from the validation set.

### Methods

***Histological assessment** -* Liver fibrosis was evaluated according to Metavir staging. Significant fibrosis was defined as Metavir stages F≥2, severe fibrosis as Metavir F≥3, and cirrhosis as F4. In the exploratory set, liver fibrosis was evaluated by two senior experts with a consensus reading at Angers, and by a senior expert at Bordeaux and Grenoble. In the FIBROSTAR study, liver fibrosis was centrally evaluated by two senior experts with a consensus reading in cases of discordance. Fibrosis staging was considered as reliable when liver specimen length was ≥15 mm and/or portal tract number ≥8 (Nousbaum JB. et al., Gastroenterol Clin Biol 2002; 26:848-78). Liver biopsy was used as the reference for the liver fibrosis evaluations by non-invasive tests.

***Fibrosis blood tests** -* The following blood tests were calculated according to published or patented formulas: Fibrotest (Castera L. et al., Gastroenterology 2005; 128:343-50), FibroMeter (Leroy V. et al., Clin Biochem 2008; 41:1368-76), Hepascore (Adams LA. et al., Clin Chem 2005; 51:1867-73), FIB-4 (Sterling RK. et al., Hepatology 2006; 43:1317-25), and APRI (Wai CT. et al., Hepatology 2003; 38:518-26). All blood assays were performed in the same laboratories of each center, or centralized in the FIBROSTAR study.

***Liver stiffness evaluation** -* Fibroscan (EchoSens, Paris, France) examination was performed by an experienced observer (>50 examinations before the study), blinded for patient data. Examination conditions were those recommended by the manufacturer (Castera L. et al., J Hepatol 2008; 48:835-47). Fibroscan examination was stopped when 10 valid measurements were recorded. Results (kilopascals) were expressed as the median and the interquartile range of all valid measurements. Fibroscan results were considered as reliable when the ratio interquartile range/result (IQR/median) was <0.21 (Lucidarme D. et al., Hepatology 2009; 49:1083-9).

### Statistical analysis

Quantitative variables were expressed as mean ± standard deviation. The diagnostic cutoffs of fibrosis tests were calculated according to the highest Youden index (sensitivity + specificity -1), unless otherwise specified.

### Fibrosis stage classifications

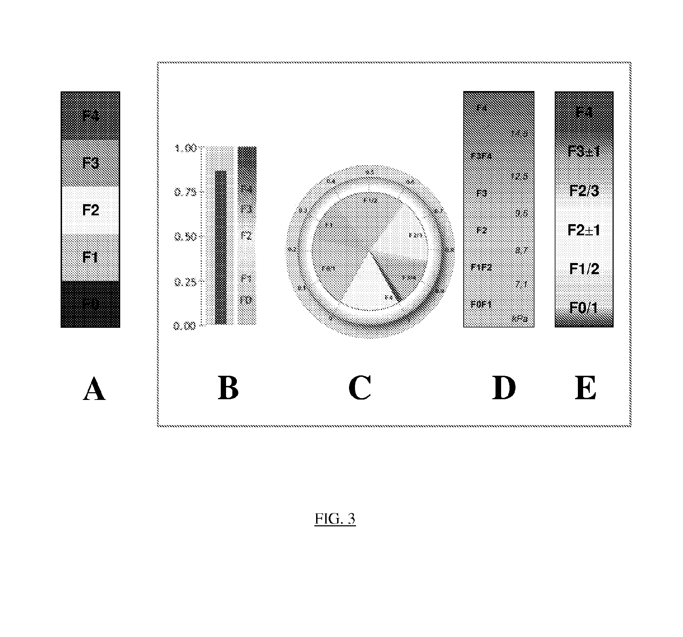

We evaluated the accuracy of Fibrotest, FibroMeter, and Fibroscan *fibrosis stage classifications* (**Figure 3****)**. Fibrotest, Fibroscan, and FibroMeter classifications were those previously published (Leroy V. et al., Clin Biochem 2008; 41:1368-76, de Ledingen V. et al., Gastroenterol Clin Biol 2008; 32:58-67, Poynard T. et al., Comp Hepatol 2004; 3:8). Fibrotest classification includes 8 classes (F0, F0/1 , F1, F1/2, F2, F3, F3/4, F4), Fibroscan classification: 6 classes (F0/1, F1/2, F2, F3, F3/4, F4), and FibroMeter classification: 6 classes (F0/1 , F1, F1/2, F2/3, F3/4, F4).

### New fibrosis stage classification

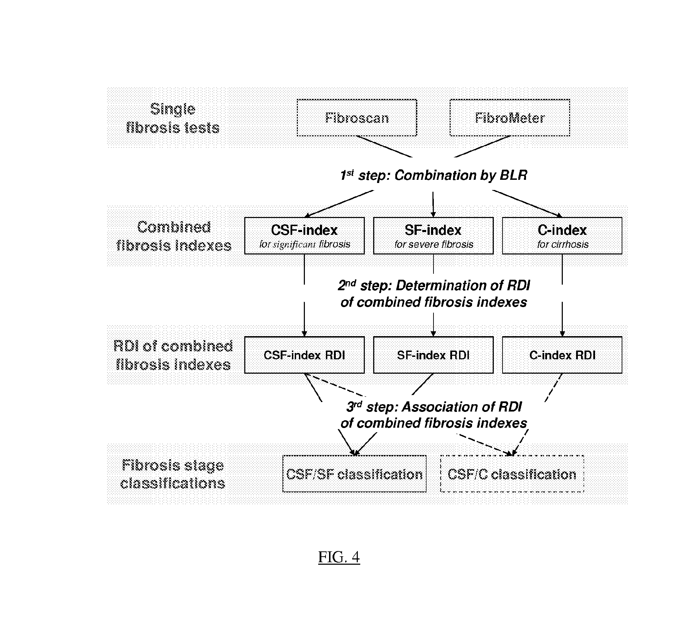

The 3-step procedure used to implement the new *fibrosis stage classification* is detailed in the **Figure 4****.**
*1^{st} step: New combined fibrosis indexes -* To identify the best combination of *single fibrosis tests* for the diagnosis of significant fibrosis, we performed a stepwise binary logistic regression repeated on 1,000 bootstrap samples in the exploratory set. Independent variables tested were the 5 blood fibrosis tests and Fibroscan. The bootstrap method consists of a repeated sampling (with replacement) from the original entire dataset, followed by a stepwise logistic regression procedure in each subsample (1,000 subsamples here). The most frequently (>50%) selected *single fibrosis tests* among the 1,000 analyses were then included in a single binary logistic regression performed in the whole population of the exploratory set. Using the regression score of this multivariate analysis, we constructed a new *combined fibrosis index* for clinically significant fibrosis called "CSF-index", ranging from 0 to 1. We also constructed *combined fibrosis indexes* for the diagnosis of severe fibrosis (SF-index) and cirrhosis (C-index) using the same process.
*2^{nd} step: Reliable diagnosis intervals -* RDIs correspond to the intervals of fibrosis test values where the individual diagnostic accuracy is considered sufficiently reliable for clinical practice. This method has been previously described (Cales P. et al., Liver Int 2008; 28:1352-62). Briefly, we first calculated the 90% negative and positive predictive value thresholds for significant fibrosis of the CSF-index. These 2 thresholds determined 3 intervals of CSF-index values: a low interval (from 0 to the 90% negative predictive value threshold) where the non-invasive diagnosis was consequently "F0/1"; a high interval (from the 90% positive predictive value threshold to 1) where the diagnosis was "F≥2"; and an intermediate interval between the two thresholds. The intermediate interval was then divided into two new intervals according to the diagnostic cut-off corresponding to the highest Youden index. In each of these two new intermediate intervals, the non-invasive diagnosis corresponded to the combined Metavir F stages having ≥90% prevalence (for example: F1/2 for the interval between the 90% negative predictive value threshold and the highest Youden index cut-off). Finally, the 4 RDI that were obtained provided ≥90% diagnostic accuracy by definition.
   We also calculated the RDIs of SF-index and C-index in the same way. Because SF-index was developed for the diagnosis of severe fibrosis, its 90% negative and positive predictive value thresholds and its highest Youden index cut-off were determined for this diagnostic target. For C-index, we calculated the thresholds for cirrhosis according to the 95% predictive values due to the clinical importance of cirrhosis diagnosis.
*3^{rd} step: New fibrosis stage classifications -* A new *fibrosis stage classification* was derived by associating RDIs for CSF- and SF-indexes. For example, if CSF-index provided a reliable diagnosis of "F≥2" and SF-index a reliable diagnosis of "F2±1", the ensuing diagnosis of the new *fibrosis stage classification* was "F2/3". Another *fibrosis stage classification* was derived by associating RDIs for CSF- and C-indexes.
   Statistical softwares were SPSS, version 17.0 (SPSS Inc., Chicago, IL, USA) and SAS 9.1 (SAS Institute Inc., Cary, NC, USA).

### RESULTS

### Patients

The exploratory and validation sets included 349 and 380 patients respectively. The characteristics of both sets are detailed in **Table 12.**

**Table 12: Patient characteristics at inclusion.**

| | **All patients** | **Set** | | |
|---|---|---|---|---|
| | | **Exploratory** | **Validation** | **p** |
| Patients (n) | 729 | 349 | 380 | - |
| Males (%) | 61.3 | 60.2 | 62.4 | 0.531 |
| Age (years) | 51.7 ± 11.2 | 52.1 ± 11.2 | 51.3 ± 11.2 | 0.347 |
| Metavir (%): | | | | <0.001 |
| F0 | 4.0 | 1.4 | 6.3 | |
| F1 | 37.7 | 30.7 | 44.2 | |
| F2 | 25.8 | 35.5 | 16.8 | |
| F3 | 17.6 | 20.6 | 14.7 | |
| F4 | 15.0 | 11.7 | 17.9 | 0.020 |
| Significant fibrosis (%) | 58.3 | 67.9 | 49.5 | <0.001 |
| Reliable biopsy (%) | 93.5 | 92.6 | 94.2 | 0.391 |
| Fibroscan result (kPa) | 10.0 ± 7.9 | 9.9 ± 8.1 | 10.1 ± 7.7 | 0.755 |
| IQR/median <0.21 (%) | 66.9 | 66.2 | 67.6 | 0.700 |

| | | | | |
|---|---|---|---|---|
| kPa: kilopascal; IQR: interquartile range | | | | |

Among the two sets, 93.5% of liver biopsies were considered as reliable.

### Development of new fibrosis stage classifications

### 1^{st} step: New combined fibrosis indexes

For each diagnostic target of liver fibrosis, Fibroscan and FibroMeter were *single fibrosis tests* the most frequently selected by the stepwise binary logistic regression repeated on the 1000 bootstrap samples. These 2 fibrosis tests were independent variables in logistic models ran in the exploratory set and thus provided 3 new *combined fibrosis indexes* for 3 diagnostic targets: CSF-index for significant fibrosis, SF-index for severe fibrosis, and C-index for cirrhosis. CSF-index had a significantly higher AUROC than its composite tests, i.e., FibroMeter or Fibroscan, in the exploratory set (**Table 13**).

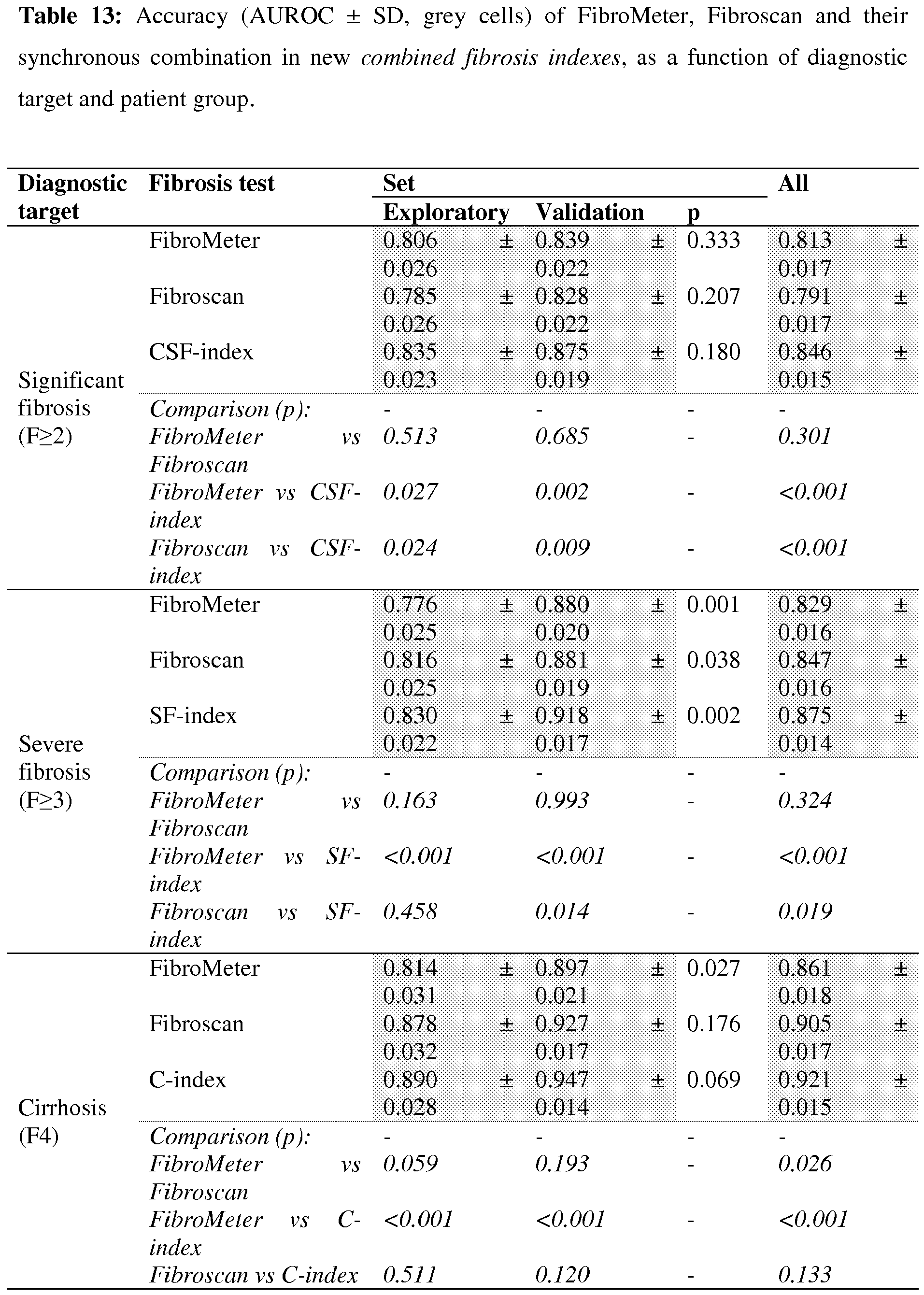

SF-index and C-index also had higher AUROCs than FibroMeter or Fibroscan in the exploratory set, but the difference was significant only with FibroMeter.

### 2^{nd} step: Reliable diagnosis intervals

*CSF-index (diagnostic target: significant fibrosis) -* CSF-index was divided into 4 reliable diagnosis intervals. The extreme intervals were the traditional intervals of ≥90% negative (NPV) or positive (PPV) predictive values for significant fibrosis. CSF-index included 9.2% of patients in the ≥90% NPV interval (CSF-index value ≥ 0 and ≤ 0.248) and 46.1% in the ≥90% PPV interval (CSF-index value ≥0.784 and ≤1). Thus, CSF-index displayed a reliable diagnosis of significant fibrosis with ≥90% accuracy in 55.3% of patients versus 33.8% with Fibroscan (p<**0.001**) and 55.6% with FibroMeter (p=1.00, **Table 14**).

**Table 14: Rate of patients included in the intervals of reliable diagnosis defined by the ≥90% negative (NPV) and positive (PPV) predictive values for significant fibrosis (Metavir F≥2) or severe fibrosis (Metavir F≥3), and the ≥95% predictive values for cirrhosis (Metavir F4), as a function of diagnostic target and fibrosis test, and according to patient group.**

| **Set** | **Fibrosis test** | **Metavir F≥2** | | **Metavir F≥3** | | **Metavir F4** | |
|---|---|---|---|---|---|---|---|
| | | **Patient s^{a}** | **Correctly classified ^{b}** | **Patients ^{a}** | **Correctly classified ^{b}** | **Patients ^{a}** | **Correctly classified ^{b}** |
| Exploratory | | | | 41.8 | 89.7 | | |
| | FibroMeter | 55.6 | 89.7 | | | 65.9 | 94.8 |
| | Fibroscan | 33.8 | 90.7 | 46.4 | 90.1 | 87.4 | 94.8 |
| | Combined | | | 49_{.}9 | 89.7 | | |
| | index ^{c} | 55.3 | 90.2 | | | 89.7 | 94.9 |
| Validation | FibroMeter | 48.8 | 72.7 | 47.0 | 94.2 | 64.2 | 97.2 |
| | Fibroscan | 38.2 | 77.0 | 46.7 | 93.5 | 85.2 | 93.2 |
| | Combined | | | 58.5 | 95.9 | 87.3 | 93.8 |
| | index ^{c} | 49.1 | 85.2 | | | | |
| All | FibroMeter | 52.3 | 82.0 | 44.3 | 92.0 | 65.1 | 95.9 |
| | Fibroscan | 35.9 | 83.6 | 46.5 | 91.8 | 86.3 | 94.0 |
| | Combined | | | 54.1 | 92.9 | 88.5 | 94.3 |
| | index ^{c} | 52.3 | 87.9 | | | | |

The indeterminate interval (between CSF-index values >0.248 and <0.784) was then divided into 2 new intervals according to the diagnostic cut-off corresponding to the maximum Youden index (0.615). 87.5% of the patients included in the lower new interval (>0.248 - <0.615) had F1/2 stages according to liver biopsy results, and 95.0% of patients included in the higher new interval (≥0.615 and <0.784) had F1/2/3 stages **(****Figure 5A****).** Finally, CSF-index provided 4 RDIs whose F classification was: F0/1, F1/2, F2±1, and F≥2. The diagnostic accuracy of these RDIs was 90.3% **(****Figure 5A****).**

FibroMeter provided the same 4 RDIs with 89.4% diagnostic accuracy (p=0.664 vs CSF-index).

*SF-index (diagnostic target: severe fibrosis) -* SF-index was also divided into 4 RDIs. The extreme intervals were the traditional intervals of ≥90% negative or positive predictive values for severe fibrosis. SF-index included 44.7% of patients in the ≥90% NPV interval (SF-index value ≥0 and ≤0.220) and 5.2% in the ≥90% PPV interval (SF-index value ≥0.870 and ≤1). Thus, SF-index displayed a reliable diagnosis of significant fibrosis with ≥90% accuracy in 49.9% of patients (**Table 14**) versus 41.8% with FibroMeter (p<**0.001**) and 46.4% with Fibroscan (p=0.235). By dividing the indeterminate interval of SF-index according to the diagnostic cut-off (maximum Youden index: 0.364), SF-index provided 4 RDI (F1±1, F2±1, F3±1, F≥3; **Figure 5B**) with 92.0% diagnostic accuracy.

Fibroscan provided the same 4 RDIs with 91.1% diagnostic accuracy (p=0.728 vs SF-index). *C-index (diagnostic target: cirrhosis) -* C-index included 87.7% of patients in the ≥95% NPV interval for cirrhosis (C-index value ≥0 and ≤0.244), and 2.0% in the ≥95% PPV interval for cirrhosis (C-index value ≥0.896 and ≤1). Thus, C-index displayed a reliable diagnosis of cirrhosis with ≥95% accuracy in 89.7% of patients (**Table 14**) versus 65.9% with FibroMeter (p<**0.001**) and 87.4% with Fibroscan (p=0.096). Dividing the indeterminate interval according to the diagnostic cut-off did not distinguish two different groups. Finally, C-index provided 3 RDIs (F≤3, F3±1, F4) with 95.1% diagnostic accuracy (**Figure 5C**).

In conclusion, by using the thresholds of 90% predictive values for significant fibrosis and the diagnostic cut-off corresponding to the maximum Youden index, CSF-index provided 4 RDIs (F0/1, F1/2, F2±1, F≥2), which provided 90.3% diagnostic accuracy. By using the same method for severe fibrosis, SF-index provided 4 RDIs (F1±1, F2±1, F3±1, F≥3) with 92.0% diagnostic accuracy. Finally, by using the thresholds of 95% predictive values for cirrhosis, C-index provided 3 RDIs (F≤3, F3±1, F4) with 95.1% diagnostic accuracy.

### 3^{rd} step: New fibrosis stage classifications

The first classification (CSF/SF classification) was derived from the association of CSF- and SF-index RDIs (**Table 15**).

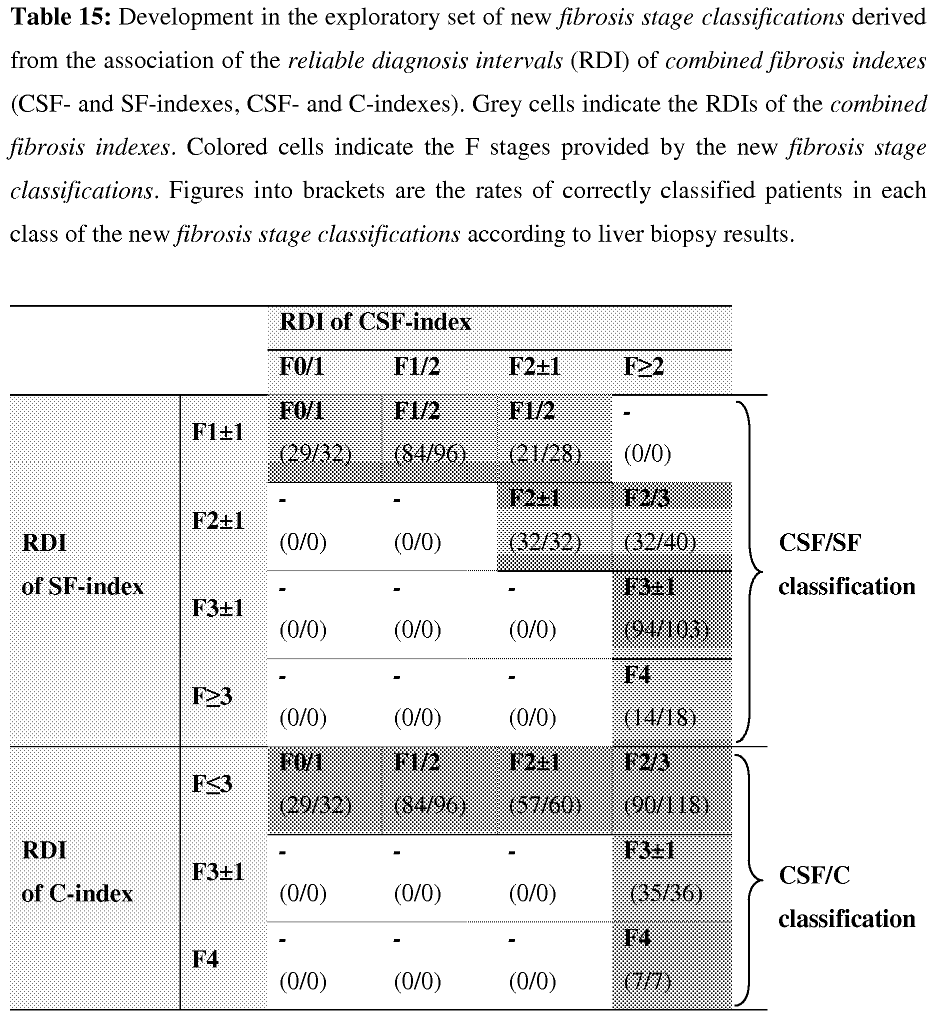

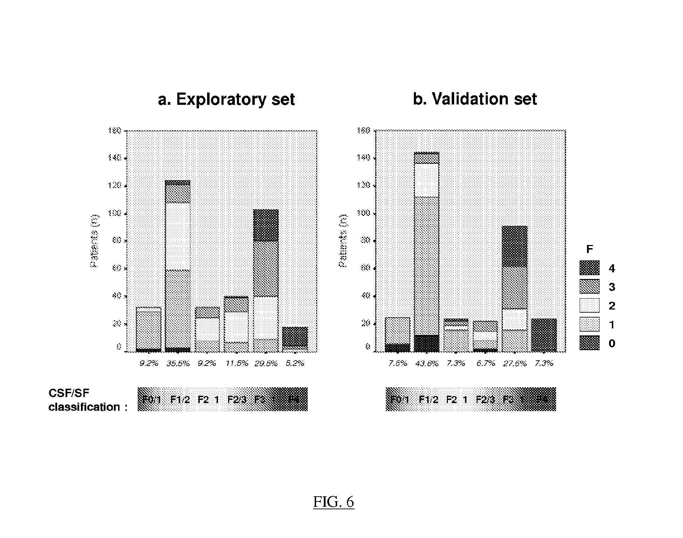

CSF/SF classification included 6 classes (F0/1, F1/2, F2±1, F2/3, F3±1, F4) and provided 87.7% diagnostic accuracy in the exploratory set (**Figure 6A**).

The second classification (CSF/C classification) was derived from CSF- and C-index RDIs (**Table 15**). CSF/C classification also included 6 classes (F0/1, F1/2, F2±1, F2/3, F3±1, F4) and provided 86.5% diagnostic accuracy (p=0.503 vs CSF/SF classification, **Table 16).**

**Table 16: Diagnostic accuracy (% of correctly classified patients) of fibrosis stage classifications as a function of patient group.**

| **Classification** | **Set** | | | |
|---|---|---|---|---|
| | **All** | **Exploratory** | **Validation** | **p ^{a}** |
| CSF/SF | 86.7 | 87.7 | 85.8 | 0.461 |
| CSF/C | 84.4 | 86.5 | 82.1 | 0.113 |
| FibroMeter | 68.7 | 67.6 | 69.7 | 0.550 |
| Fibroscan | 58.7 | 54.4 | 63.3 | 0.020 |
| Fibrotest | 38.8 | 33.5 | 43.9 | 0.005 |

### Association of combined fibrosis indexes RDIs or single fibrosis tests RDIs?

As previously shown, the accuracies of RDIs from *combined fibrosis indexes* and their composite *single fibrosis tests* were not significantly different (i.e., the FibroMeter RDIs for significant fibrosis vs that of CSF-index, and the Fibroscan RDIs for severe fibrosis vs that of SF-index). Therefore, we implemented a third classification (FM/FS classification) that was derived from the FibroMeter RDIs for significant fibrosis and the Fibroscan RDIs for severe fibrosis. Results of FibroMeter and Fibroscan RDIs were discordant in 2 patients, who thus had indeterminate diagnoses. FM/FS classification ultimately included 7 classes (F0/1, F1, F1/2, F2, F2/3, F3±1, F4) and provided 82.8% diagnostic accuracy (p=0.006 vs CSF/SF classification). However, diagnostic accuracy of FM/FS classification dramatically decreased to 69.4% in the validation set (p<0.001 vs CSF/SF and CSF/C classifications).

### Validation of the new fibrosis stage classifications

The diagnostic accuracies of CSF-index, SF-index, and C-index RDIs were not significantly different between the exploratory and the validation sets, with respectively: 90.3% vs 86.7% (p=0.142), 92.0% vs 91.5% (p=0.827), and 95.1% vs 94.5% (p=0.731). Similarly, diagnostic accuracies of CSF/SF and CSF/C classifications were not significantly different between the 2 sets (**Table 16**).

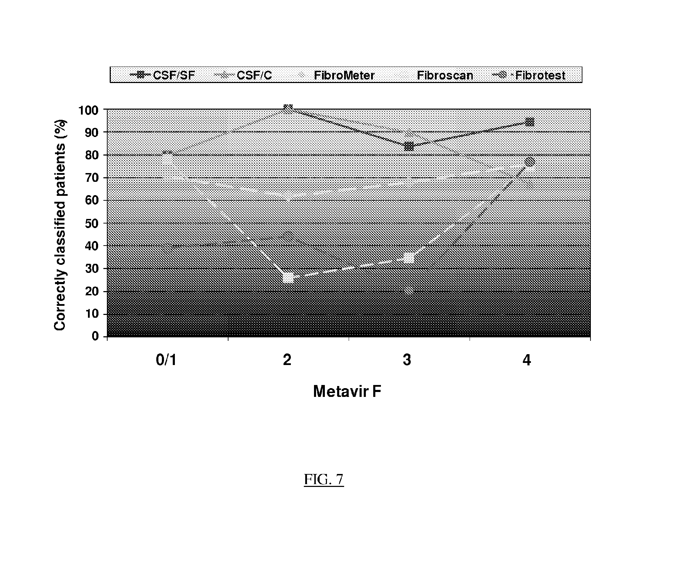

In the validation set, CSF/SF classification provided a significantly higher diagnostic accuracy (85.8%) than CSF/C classification and those of *single fibrosis tests* (p<0.008, **Table 16**). **Figure 6B** shows the proportion of Metavir fibrosis stages as a function of CSF/SF classification. According to diagnostic accuracy in the validation set, classification ranking was: CSF/SF > CSF/C > FibroMeter > Fibroscan > Fibrotest (**Table 16**). **Figure 7** shows the diagnostic accuracy of each *fibrosis stage classification* as a function of Metavir fibrosis stage in the validation set. Among *single fibrosis tests,* FibroMeter provided the most homogeneous profile with no significant differences among histological fibrosis stages (p=0.352). The new CSF/SF and CSF/C classifications provided better profiles than those of *single fibrosis tests.* However, the rate of well classified patients among cirrhotic patients was significantly higher with CSF/SF classification (94.5%) than with CSF/C classification (67.3%, p<0.001).

### Influencing factors

In the whole study population, we performed a stepwise binary logistic regression including age, sex, biopsy length, Metavir F, and IQR/median as independent variables. The rate of well classified patients by CSF/SF classification was independently associated with the ratio IQR/median (1^{st} step, exp(β)=0,322), Metavir F (2^{nd} step, exp(β)=1.370), and age (3^{rd} step, exp(β)=0.976)

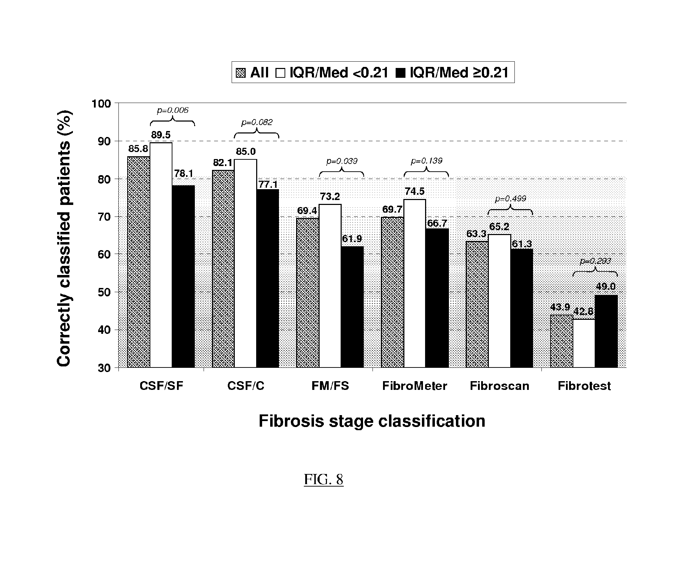

In the validation set, CSF/SF classification provided 89.5% diagnostic accuracy in patients with IQR/median <0.21 versus 78.1% in patients with IQR/median ≥0.21 (p=0.006). In the subgroup of patients with IQR/median <0.21, CSF/SF classification had the highest diagnostic accuracy (p=0.006 vs other classifications, **Figure 8**).

## Claims

1. Non-invasive method for assessing the presence and/or severity of a lesion in an organ of an animal, including a human, said method comprising carrying out at least one non-invasive test resulting in a score/value, and positioning the at least one score/value in a class of a detailed classification based on population percentiles, or in a reliable diagnostic interval (RDI), to be crossed with another RDI for a final positioning of both RDIs in a class.

2. Non-invasive method according to claim **1**, wherein the organ is the liver and the detailed classification is a detailed fibrosis classification and/or a detailed necrotico-inflammatory activity classification.

3. Non-invasive method according to claim **1** or claim **2**, wherein the animal, including a human is at risk of suffering or is suffering from a condition selected from the group consisting of a liver impairment, chronic liver disease, a hepatitis viral infection especially an infection caused by hepatitis B, C or D virus, an hepatoxicity, a liver cancer, a steatosis a non alcoholic fatty liver disease (NAFLD), a non-alcoholic steatohepatitis (NASH), an autoimmune disease, a metabolic liver disease and a disease with secondary involvement of the liver.

4. Non-invasive method according to anyone of claims **1** to **3**, wherein the non-invasive test comprises at least one combination score, obtained by synchronous binary combination of at least one biomarker, at least one clinical marker, at least one data resulting from a physical method and/or at least one score.

5. Non-invasive method according to claim **4**, wherein said combination score is selected from the group comprising ELF, FIBROSpect™, APRI, FIB-4, Hepascore, Fibrotest™, CirrhoMeter™ and FibroMeter™, preferably said non-invasive score is a FibroMeter^{3G}.

6. Non-invasive method according to claim **4**, wherein said physical method is selected from the group comprising medical imaging data, preferably is selected from the group comprising ultrasonography, especially Doppler-ultrasonography, elastometry ultrasonography and velocimetry ultrasonography, such as, for example, Fibroscan, ARFI, VTE; IRM; and MNR, especially MNR elastometry or velocimetry, more preferably the physical method is Fibroscan.

7. Non-invasive method according to anyone of claims **1** to **6**, said method comprising carrying out at least one non-invasive test resulting in a score/value, and positioning the at least one score/value in a class of a detailed fibrosis and/or activity classification based on percentiles, wherein said classification is based on the discretization of the scores of a reference population into at least 10 percentiles of 10% of the population, preferably into at least 20 percentiles of 5% of the population, more preferably into 40 percentiles of 2.5% of the population.

8. Non-invasive method according to anyone of claims **1** to **6**, said method comprising the steps of performing at least two non-invasive tests resulting in at least two scores/values.

9. Non-invasive method according to claim **8**, wherein said at least two non-invasive tests are FibroMeter™ and Fibroscan.

10. Non-invasive method according to claim **8** or claim **9**, comprising the steps of:
- combining the scores/values obtained from two non-invasive tests in at least two binary logistic regressions to obtain at least two indexes,
- positioning each index on a RDI, wherein the position of RDI has been determined from a reference population,
- combining both RDIs according to a double entry table of RDIs showing combined classes, and
- positioning the RDIs in a combined class.

11. A device carrying out the non-invasive method according to anyone of claims **1** to **10**.

12. The device according to claim **1**1, being a meter reflecting the detailed stage classification, such as, for example, the new detailed fibrosis stage classification or the new detailed necrotico-inflammatory activity grade classification, based on the discretization of the scores of a reference population into percentiles.

13. The device according to claim **11,** being a meter reflecting the detailed stage classification, such as, for example, the new detailed fibrosis stage classification or the new detailed necrotico-inflammatory activity grade classification, based on the combination of reliable diagnostic intervals.
